# EUROPEAN PATENT SPECIFICATION

(11) **EP 2 878 308 B1**
(45) Date of publication and mention of the grant of the patent: **31.10.2018**
(21) Application number: 14004060.1
(22) Date of filing: 02.12.2014
(51) Int. Cl.: A61K 39/395, A61K 39/44, C07K 16/22, C07K 16/28, A61K 38/18, A61P 37/06

(54) **Agents and methods for the suppression of T cell activation**
Mittel und Verfahren zur Unterdrückung der T-Zell-Aktivierung
Agents et procédés pour la suppression de l'activation des lymphocytes T

(30) Priority: 02.12.2013 EP 13005586
(43) Date of publication of application: 03.06.2015
(73) Proprietor: Harder, Thomas, 35510 Butzbach (DE)
(72) Inventor: Harder, Thomas, 39108 Magdeburg (DE); Reinhold, Dirk, 39108 Magdeburg (DE); Guttek, Karina, 39108 Magdeburg (DE); Schraven, Burkhart, 39175 Biederitz (DE)
(74) Representative: Müller-Boré & Partner Patentanwälte PartG mbB

(56) References cited:
- WO-A1-2011/078990
- WO-A2-02/055098
- WO-A2-2009/144457
- US-A1- 2011 268 716
- L. L. MOLINERO ET AL: "High TCR Stimuli Prevent Induced Regulatory T Cell Differentiation in a NF- B-Dependent Manner", THE JOURNAL OF IMMUNOLOGY, vol. 186, no. 8, 16 March 2011 (2011-03-16), pages 4609-4617, XP055099632, ISSN: 0022-1767, DOI: 10.4049/jimmunol.1002361

## Description

The present invention relates to a bifunctional agent that is suitable for suppressing the activation of T lymphocytes, said agent comprising antibodies, antibody fragments, or antibody mimetics that are capable of activating the T cell receptor (TCR) and a transforming growth factor β receptor (TGFβR) of said T lymphocytes. The present invention further relates to a method for the suppression of the activation of T lymphocytes, comprising the step of incubating said lymphocytes with a bifunctional agent of the present invention.
A central mechanism to ensure a healthy balance of immune reactions is the control of T lymphocyte (T cell) activity. T cell receptor (TCR) signaling is the key to the activation of resting T lymphocytes, triggering proliferation of the cells and respective effector functions during an immune response. Activation, proliferation and effector functions are normally subjected to strict control mechanisms which prevent unwanted T cell activation. In the course of these highly complex control mechanisms, the activity of T lymphocytes is inhibited by the cytokine transforming growth factor beta (TGFβ).
TGFβ has pleiotropic roles in steering differentiation and homeostasis of cells, tissues, and organs. TGFβ1 is a versatile regulator of T cell immune responses and guides CD4⁺ T lymphocytes toward specific activities of the T_{H}17 and Treg phenotypes. Additionally, TGFβ signals restrain T cell activities and thereby directly function in immune homeostasis. Functions of TGFβ1 in safeguarding a healthy balance of the immune system were highlighted by severe multiorgan inflammatory disease of TGFβ1-deficient mice. Dysfunction of TGFβ1-mediated T cell balance is associated with autoimmune disorders and thus molecular principles of TGFβ-regulation of T cell activities are of important medical interest.

TGFβ signals are transduced by TGFβ receptors (TGFβR) I and II which catalyze serine/threonine phosphorylation of SMAD proteins. Phosphorylated SMADs cooperate with various transcription factors to activate cell-specific gene transcription programs. TGFβ receptors, cytoplasmic and nuclear factors, and chromatin structure determine context-dependent cellular outcomes of these canonical TGFβ signaling cascades. Additional non-canonical TGFβ signaling pathways exist, many of which are initiated at the platform of the plasma membrane for example by targeting PI3K/AKT pathway and Rho-GTPase signaling cascades.

Under physiological, resting conditions TGFβ is held in an inactive latent configuration in a complex with Latency Associated Peptide (LAP) which hinders TGFβ cytokine binding to TGFβ receptors. Latent TGFβ gets activated when active cytokine is released from the TGFβ-LAP complex. Various mechanisms mediate activation of TGFβ: These include specific binding of proteins like thrombospondin to LAP, LAP proteolytic cleavage, αv integrin-binding and cytoskeletal force along membrane-bound TGFβ-LAP complex.

T cell antigen receptor (TCR), upon interaction with a cognate peptide-MHC ligand, transduces key signals which activate resting T lymphocytes to proliferate and perform immune effector functions. These signals are propagated to the T cell interior via tyrosine kinase activation and induction of signaling complexes which mediate intracellular signaling reactions and respective T cell responses. A costimulatory second signal is mediated by surface protein CD28 on engagement with CD80/86 antigens on antigen-presenting cells and is required for full activation of resting T lymphocytes to proliferate and perform effector functions like secretion of cytokines.

TGFβ1-LAP is presented on the surface of CD4⁺ CD25⁺ T cells and was proposed to exert regulatory functions upon contact with effector T cells. On FoxP3⁺ regulatory T cells (Tregs), TGFβ1-LAP is bound to trans-plasma membrane protein GARP (glycoprotein A repetitions predominant protein). siRNA-mediated depletion of GARP in human Tregs reduced, but did not abolish, suppressive activity of FoxP3⁺ Tregs *in vitro,* indicating a contribution of TGFβ1 surface presentation as well as additional functions to regulatory activities of Tregs. A distinct population of FoxP3⁺ human Tregs expresses MHC class II and was shown to specifically mediate early suppression of T cell activation directly upon cell/cell contact.

TCR activation and TGFβ1-signals may likewise act together at the immunological synapse between CD4⁺ T cells and antigen-presenting dendritic cells (DC) in order to induce Treg and T_{H}17 activities. These DCs need to express matching MHC class II to activate TCR and αv integrins which present TGFβ1 to T cells.

In this context, WO 2011/078990 A1 describes hydrogels comprising hyaluronan and T cell inducing agents which activate T cells to adopt a specific T cell phenotype. Further WO 2009/144457 A2 describes biomaterials having a latent form of TGFbeta-family of growth factors immobilized thereon. These macroscopic biomaterials are designed as transplantable material and may be used in medicine, such as in tissue regeneration or repair.

In cases where control mechanisms for the prevention of unwanted T lymphocyte activity fail, a number of pathological conditions and diseases such as inflammatory diseases, autoimmune diseases, e.g. diabetes mellitus type I or multiple sclerosis (MS), bowel diseases such as Morbus Crohn, and transplant rejection can develop. Therapy of respective diseases and conditions has been attempted by administration of activated TGFβ to suppress excessive T lymphocyte activity. However, such trials failed consistently and had to be aborted, since exogenous administration of TGFβ leads to severe side effects and complications. This is likely due to the fact that TGFβ regulates a number of further functions in the human body.

Therefore, there still exists a great need for means and strategies that allow for the specific control of T lymphocyte activation and activity. This would in turn allow for new therapeutic strategies for the prevention and treatment of a number of diseases and conditions such as e.g. diabetes mellitus type I or multiple sclerosis (MS), and transplant rejection.

Accordingly, the technical problem underlying the present invention is to provide compounds and methods for the suppression of T lymphocyte activation and activity.

The solution to the above technical problem is achieved by the embodiments characterized in the claims.

The present invention relates to the following items:
1. A bifunctional agent that is suitable for suppressing the activation of T lymphocytes, said agent having the general structure 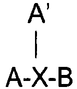 wherein
   - A: is an antibody, antibody fragment or antibody mimetic directed against CD3,
   - A': is an antibody, antibody fragment, or antibody mimetic directed against CD28,
   - B: is an antibody, antibody fragment, or antibody mimetic directed against latency-associated peptide (LAP) of the TGFβ-LAP Small Latent Complex, wherein said antibody, antibody fragment or antibody mimetic is loaded with exogenous TGFβ-LAP complexes, and
   - X: is selected from the group consisting of a bead, a magnetic bead, a polymeric matrix, and a solid support,
   wherein said agent comprises more than one individual molecule of the types A, B, and A',
   wherein said antibodies, antibody fragments or antibody mimetics and the TGFβ-LAP complexes are present in a ratio of 0.5 to 1.5 parts A, 2.5 to 7.5 parts A', 2.5 to 7.5 parts B, and 0.02 to 10 parts TGFβ-LAP complexes, and wherein said antibody mimetics are selected from the group consisting of single-chain variable fragments (scFv), single-domain antibodies, affibodies, anticalins, DARPins, monobodies, and peptide aptamers.
2. The agent according to item 1, wherein said TGFβ-LAP complexes are TGFβ1-LAP complexes.
3. The agent according to item 1 or item 2, wherein X is a bead.
4. The agent according to item 3, wherein a single bead comprises 1 × 10⁷ to 1 × 10⁸ individual TGFβ-LAP complexes.
5. The agent according to any one of items 1 to 4 for use in medicine.
6. The agent according to any one of items 1 to 4 for use in the prevention or treatment of a disease or condition that is associated with excessive and/or uncontrolled T lymphocyte activation.
7. The agent for use according to item 6, wherein the disease or condition that is associated with excessive and/or uncontrolled T lymphocyte activation is a disease or condition selected from the group consisting of autoimmune diseases, such as diabetes mellitus type I, multiple sclerosis (MS), and rheumatoid arthritis; inflammatory diseases, such as inflammatory bowel diseases, Morbus Crohn, and ulcerative colitis; transplant rejection; allograft rejection; graft-versus-host disease; and allergies.
8. An *in vitro* method for the suppression of the activation of T lymphocytes, said method comprising the step of incubating the T lymphocytes with an agent according to any one of items 1 to 4.
9. The method according to item 8, wherein the suppression is a complete blocking of T lymphocyte activation.

Generally, described herein is a bifunctional agent that is suitable for suppressing the activation of T lymphocytes, said agent having the general structure

A-X-B

wherein
- A: is a polypeptide that is capable of directly or indirectly activating the T cell receptor (TCR) of said T lymphocytes,
- B: is a polypeptide that is capable of directly or indirectly activating a transforming growth factor beta receptor (TGFβR) of said T lymphocytes, and
- X: is selected from the group consisting of a bond, a linker, a peptide, a polypeptide, a bead, a magnetic bead, a polymer, a polymeric matrix, and a solid support.

The term "bifunctional agent" as used herein relates to an agent that combines two functions in physical proximity in one compound or structure, *i.e.* the function of being capable of activating the TCR, and the function of being capable of activating a TGFβR.

The term "suppression of activation of T cells" as used herein relates to any down-regulation of T cell activation and/or T cell activity. Preferably, said suppression is a complete or essentially complete blocking of T cell activation and/or T cell activity.

The term "direct or indirect activation" as used herein relates to activation by an immediate action on the TCR or TGFβR (direct activation), or activation via an intermediate agent or mechanism (indirect activation).

Polypeptides that are capable of activating the TCR of T lymphocytes are not particularly limited and are known in the art. Further, polypeptides that are capable of activating a transforming growth factor beta receptor (TGFβR) of T lymphocytes are not particularly limited. In this context, the term "transforming growth factor beta receptor (TGFβR)" as used herein relates to receptors of all three subtypes, *i.e.* type I, type II, and type III TGFβR.

In preferred embodiments, the polypeptides A and/or B are selected from the group consisting of antibodies, antibody fragments, and antibody mimetics. In this context, antibody fragments include Fab fragments, F(ab')₂ fragments, and Fab' fragments, and antibody mimetics include single-chain variable fragments (scFv); single-domain antibodies, affibodies, anticalins, DARPins, monobodies, and peptide aptamers.

However, polypeptides that are capable of activating the TCR of T lymphocytes include also for example major histocompatibility complex (MHC) molecules, including MHC class I and MHC class II molecules, that are loaded with an antigenic peptide, respective MHC molecules having an antigenic peptide covalently attached thereto, or fragments of said MHC molecules. These molecules include monomeric MHC molecules or fragments thereof, as well as multimerized MHC molecules or fragments thereof, e.g. MHC tetramers, multimers and other constructs known in the art. Moreover, polypeptides that are capable of activating the TCR of T lymphocytes include for example antibodies, antibody fragments, and antibody mimetics as defined above, which specifically bind to e.g. the TCR, in particular the antigen/MHC-recognizing region of the TCR, the TCR-coreceptor CD3, or CD28. In a preferred embodiment, A is an antibody or antibody fragment directed against CD3 or directed against CD28. In another preferred embodiment, A is an antibody or antibody fragment directed against the antigen/ MHC-recognizing region of the TCR. In another preferred embodiment, A is a monomeric or multimeric MHC molecule or fragment thereof as defined above.

In this context, described herein are agents that are directed against T lymphocytes in general, e.g. when using an antibody or antibody fragment directed against CD3 as the polypeptide that is capable of activating the TCR, as well as agents that are directed against T lymphocytes having a particular specificity, e.g. when using an antibody or antibody fragment directed against the antigen/ MHC-recognizing region of the TCR, or when using a particular MHC molecule, as the polypeptide that is capable of activating the TCR. In this manner, the activation of particular T lymphocyte populations, e.g. autoreactive T lymphocytes that mediate a particular autoimmune disease, can be specifically and selectively suppressed.

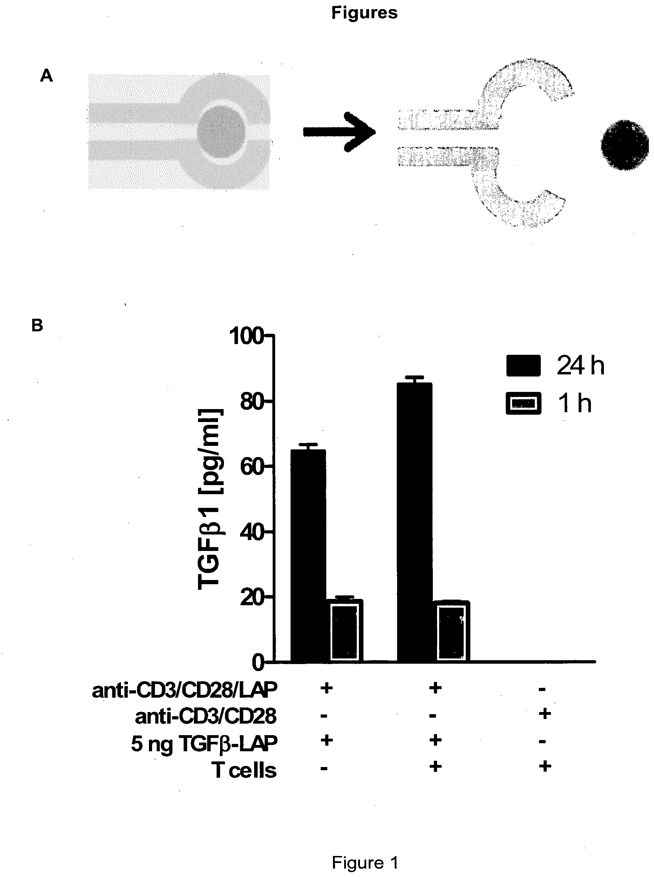

Polypeptides that are capable of activating a TGFβR of T lymphocytes include for example TGFβ, as well as antibodies, antibody fragments, and antibody mimetics as defined above, which specifically bind to the TGFβR. In a particular embodiment, the polypeptide that is capable of activating a TGFβR of T lymphocytes is an antibody, antibody fragment, or antibody mimetic as defined above which specifically binds to Latency Associated Peptide (LAP) of the Small Latent TGFβ-LAP Complex (SLC) (Fig. 1A). Endogenous SLC is always present in human serum in sufficient amounts to be captured by said antibody, antibody fragment, or antibody mimetic. In this embodiment, the bifunctional agent described herein binds to SLC via LAP. Subsequently, active TGFβ cytokine is gradually released from bifunctional agent-bound SLC (Fig. 1A, B) and activates TGFβR in physical proximity of the TCR-activating polypeptide A.
As described herein, X can be selected from the group consisting of a bond, *i.e.* a chemical bond, a linker, a peptide, a further polypeptide, a bead, in particular a magnetic bead, a polymer, a polymeric matrix, and a solid support. Respective linkers, peptides, polypeptides, beads, in particular magnetic beads, polymers, polymeric matrixes, and solid supports that can be used in the context of the present disclosure are not particularly limited and are known in the art.

The bifunctional agent described herein comprises a further component A' which is bound to X, wherein A' is a further polypeptide that is capable of activating the TCR of said T lymphocytes. In this particular embodiment, the agent has the general structure 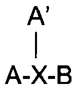 wherein A, X, B, and A' are as defined above.

A is an antibody or antibody fragment directed against CD3, and A' is an antibody or antibody fragment directed against CD28, or vice versa. In another preferred embodiment, A is an antibody or antibody fragment directed against the antigen/MHC-recognizing region of the TCR, and A' is an antibody or antibody fragment directed against CD3, or vice versa.

It should be noted that within the scope of the above definitions of the bifunctional agent described herein, in case X is a linker, a peptide, a polypeptide, a bead, a magnetic bead, a polymer, a polymeric matrix, or a solid support, *i.e.* in case where X is not merely a bond, the agent described herein can comprise more than one individual molecule of the types A, B, and/or A'. For example, X may be a magnetic bead or a solid support that is loaded with a multitude of individual molecules of e.g. an antibody directed against CD3, a multitude of individual molecules of e.g. an antibody directed against CD28, and a multitude of individual molecules of e.g. an antibody directed against an TGFβR. Such embodiments are expressly intended to fall within the scope of the above definitions of the bifunctional agent described herein.
The following describes particularly preferred embodiments of the agent described herein relating to the targeting of TGFβ, preferably TGFβ1, to the T lymphocytes.
In one of these embodiments, B, *i.e*., the polypeptide that is capable of directly or indirectly activating a TGFβR of T cells, is an antibody, antibody fragment or antibody mimetic as defined above, directed against LAP. As indicated above, endogenous TGFβ-LAP complexes (SLC) are always present in human serum in sufficient amounts to be captured by said antibody, antibody fragment, or antibody mimetic. In this embodiment, the bifunctional agent described herein binds to endogenous SLC via LAP. Subsequently, active TGFβ cytokine is gradually released from bifunctional agent-bound SLC (Fig. 1A, B) and activates TGFβR in physical proximity of the TCR-activating polypeptide A. However, in an alternative and particularly preferred embodiment, the antibody, antibody fragment or antibody mimetic directed against LAP is loaded with exogenous TGFβ-LAP complexes, preferably TGFβ1-LAP complexes, that gradually release the TGFβ in physical proximity of the TCR-activating polypeptide A.

In particular, it is preferred that the bifunctional agent described herein comprises the further component A' which is bound to X, wherein A' is a further polypeptide that is capable of activating the TCR of T lymphocytes. In this particular embodiment, the agent has the general structure 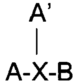 wherein A, X, B, and A' are as defined above.
Preferably, A is an antibody, antibody fragment or antibody mimetic directed against CD3, and A' is an antibody, antibody fragment or antibody mimetic directed against CD28, or vice versa.

Further, it is preferred that X is a bead, e.g. a microbead, wherein suitable beads are not particularly limited and are known in the art. Such beads include for example beads having a diameter of e.g. 2.8 µm, being coated with e.g. protein G for the coupling of A, A', and B. Respective beads are commercially available e.g. under the trademark name "Dynabeads® Protein G" (Novex, Life Technologies).

In a particularly preferred embodiment, X is a bead, the agent described herein comprises a multitude of individual molecules of A, A', and B, A is an antibody, antibody fragment or antibody mimetic directed against CD3, and A' is an antibody, antibody fragment or antibody mimetic directed against CD28, or vice versa, and B is an antibody, antibody fragment or antibody mimetic as defined above directed against LAP, wherein said antibody, antibody fragment or antibody mimetic is loaded with exogenous TGFβ-LAP complexes, preferably TGFβ1-LAP complexes. In this particularly preferred embodiment, it is preferred that the antibodies, antibody fragments or antibody mimetics and the TGFβ-LAP complexes are present on the bead in a ratio of about 1 part antibody, antibody fragment or antibody mimetic directed against CD3, about 5 parts antibody, antibody fragment or antibody mimetic directed against CD28, about 5 parts antibody, antibody fragment or antibody mimetic directed against LAP, and about 0.02 to about 100, preferably about 0.02 to about 10, more preferably about 0.02 to about 1 parts TGFβ-LAP complex. In this particular embodiment, the term "about" is intended to allow for a deviation of the specified value by ± 50%, ± 30%, ± 20%, ± 15%, ± 10%, ± 7.5%, ± 5%, ± 3%, ± 2%, or ± 1%. For example, the term "about 5 parts" as used herein is expressly intended to encompass the ranges of 2.5 to 7.5 parts, 3.5 to 6.5 parts, 4 to 6 parts, 4.25 to 5.75 parts, 4.5 to 5.5 parts, 4.625 to 5.375 parts, 4.75 to 5.25 parts, 4.85 to 5.15 parts, 4.9 to 5.1 parts, and 4.95 to 5.05 parts. Further, it is preferred that a single bead comprises 5 × 10⁶ to 1.25 × 10⁸, preferably 1 × 10⁷ to 1 × 10⁸, more preferably 3 × 10⁷ to 1 × 10⁸, more preferably 5 × 10⁷ to 8 × 10⁷, and most preferably 6 × 10⁷ to 7 × 10⁷ individual TGFβ-LAP complexes, wherein any of these absolute numbers of TGFβ-LAP complexes can be used in combination with any of the above ratios with respect to antibodies, antibody fragments or antibody mimetics directed against CD3, CD28, and LAP.

In a particular embodiment described herein, the bifunctional agent described herein is for use in medicine. In another particular embodiment, the bifunctional agent described herein is for use in the prevention or treatment of a disease or condition that is associated with excessive and/or uncontrolled T lymphocyte activation. Respective diseases and conditions are not particularly limited and are known in the art. They include autoimmune diseases, such as diabetes mellitus type I, multiple sclerosis (MS), and rheumatoid arthritis; inflammatory diseases, such as inflammatory bowel diseases, Morbus Crohn, and ulcerative colitis; transplant rejection; allograft rejection; graft-versus-host disease; and allergies.

Described herein is a method for the suppression of the activation of T lymphocytes, said method comprising the step of simultaneously activating the T cell receptor (TCR) and a transforming growth factor beta receptor (TGFβR) of said T lymphocytes.

In this aspect, the terms "suppression of activation of T cells" and "transforming growth factor beta receptor (TGFβR)" are as defined above.

In the method described herein, the TCR and a TGFβR are preferably activated in immediate proximity with each other on the surface on the T lymphocyte surface.

According to the present disclosure, the suppression of the activation of T lymphocytes is preferably reversible. In particular, after withdrawal of the stimulus that provides the simultaneous activation of the TCR and a TGFβR, the T lymphocytes return back to their original levels of activation/activity.

In a preferred embodiment of the method described herein, said method comprises the step of incubating the T lymphocytes with a bifunctional agent as defined above. Methods and parameters for a respective incubation are not particularly limited and are known in the art.

Preferably, the methods described herein as defined above are *in vitro* methods, *i.e.* said methods are not performed on the human or animal body.

The present invention allows for new strategies for the efficient and specific suppression of T lymphocyte activation and activity, either in a global or population-specific manner. With the help of the bifunctional agents described herein, TCR and TGFβR are simultaneously activated in immediate proximity of each other on the surface of the T lymphocyte. This leads to a complete but reversible suppression of T lymphocyte activation and activity. In particular, the present invention allows for the efficient and specific targeting of TGFβ activity to T lymphocyte activation and activity, thereby advantageously reducing systemic TGFβ side effects and complications. Moreover, the reversibility of the suppression enhances practicality and tolerance of respective pharmaceuticals for the patient.

The bifunctional agents described herein have the potential to develop into versatile and flexible means to prevent and cure conditions and diseases that are mediated by an excessive and overshooting T cell activation. Such diseases and conditions include a wide range of inflammatory diseases, autoimmune diseases, and transplant rejection effects. They include prominent examples such as diabetes mellitus type I, multiple sclerosis (MS), and graft-versus-host disease.

Pleiotropic action and consequent severe side effects preclude use of active TGFβ in medical applications for the treatment of pathological conditions connected to unwanted T cell activity. The present invention provides a new strategy to circumvent these difficulties, *inter alia* by selective targeting of TGFβ1 to TCR activation plasma membrane domains in order to specifically silence T cell responses. Bifunctional biologicals which link functions of TCR activation and TGFβRI and II activation proximally in the plasma membrane promise applications in therapy of disease associated to unbalanced, unwanted T cell activity in autoimmunity, allergy or transplant rejection.

Simultaneous presentation of TGFβ1 and TCR-activating ligands on cell surfaces is a recurring motif in modulation of T cell activities. The present invention provides *inter alia* defined ligand-coated beads as artificial surfaces to present activating stimuli to resting human T cells *in vitro:* Simultaneous presentation of TGFβ1 and TCR/CD28-signals in common T cell plasma membrane signaling domains stringently and reversibly held back activation responses of T cells. These findings identified lateral plasma membrane compartmentalization of TGFβ1 signaling as cell biological context under which T cell activation responses are attenuated and suggest new strategies for treatment of disease and pathological conditions which are caused by unwanted T cell activity.

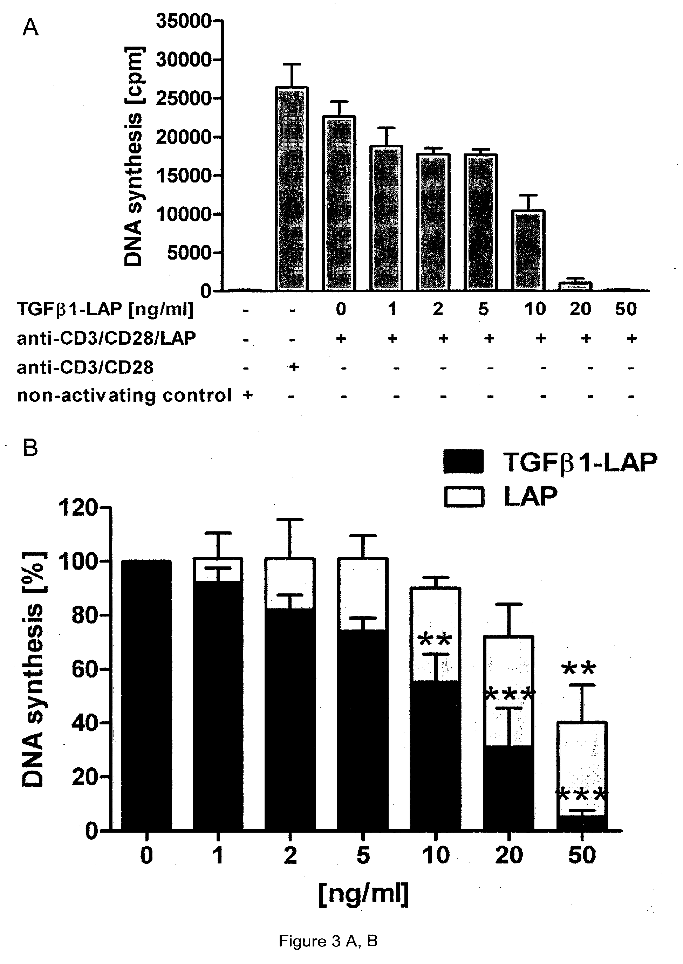

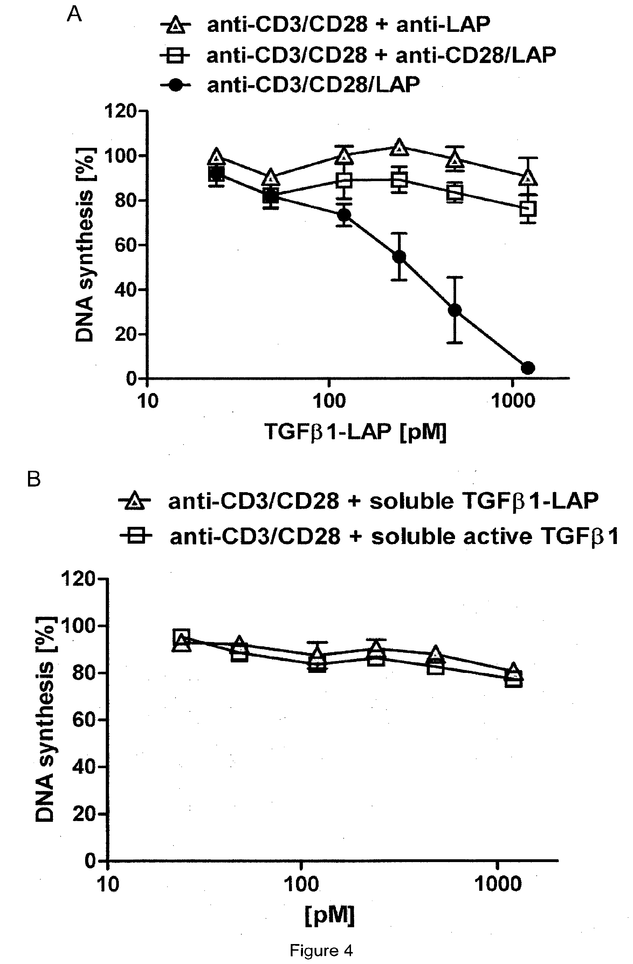

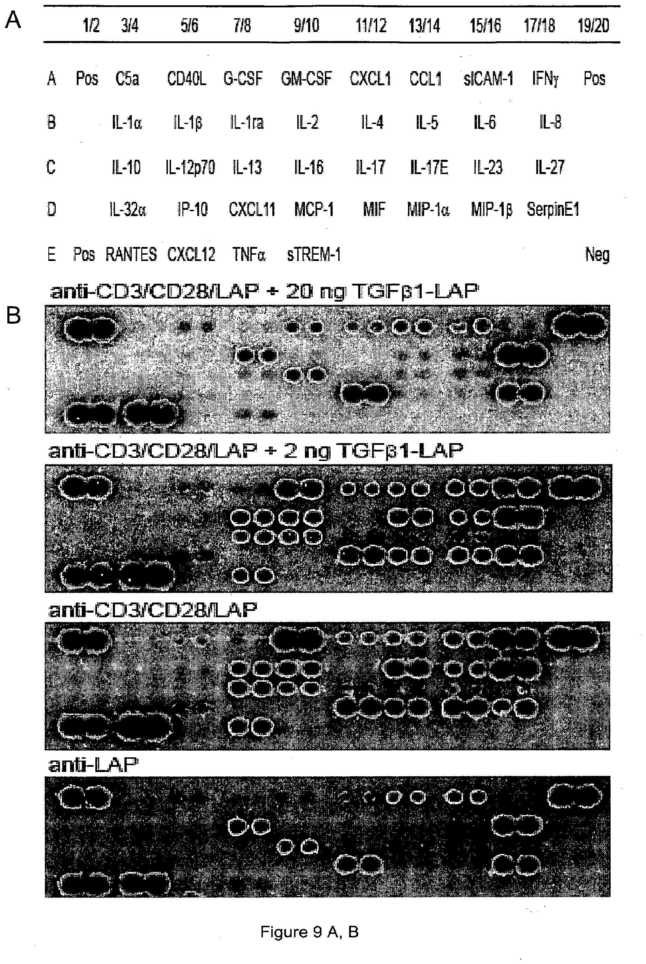

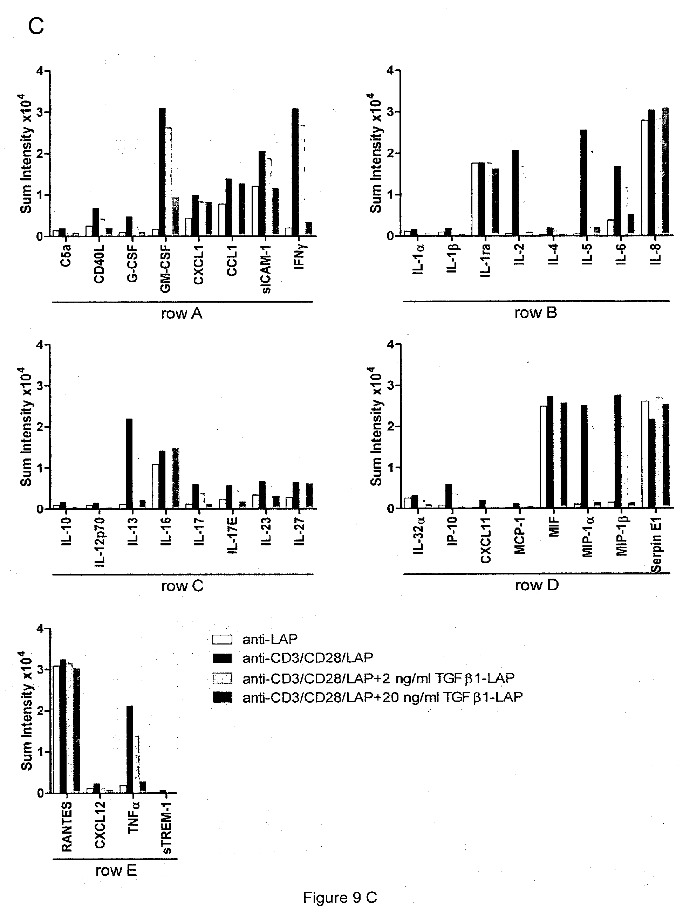

The figures show:
Figure 1:
   TGFβ cytokine is gradually released from TGFβ-LAP-coated beads at 37°C.
   A: Schematic of the Small Latent Complex (SLC) consisting of TGFβ cytokine (dark gray circle) and LAP (Latency Associated Peptide, light gray). Release of active TGFβ cytokine from SLC is indicated.
   B: 3 µg anti-CD3/CD28/LAP beads were loaded with 5 ng SLC/TGFβ-LAP, transferred into 100 µl AIMV medium in commercial TGFβ-ELISA plates (BD Life Technologies), and incubated for 1 h (gray bars) and 24 h (black bars) at 37°C. Spontaneous release of TGFβ cytokine into the aqueous supernatant was demonstrated by these ELISA assays and presence of T cells elicited a ∼1.3 fold increase at the 24 h time point. No TGFβ cytokine was released by anti-CD3/CD28 bead-activated T cells.
Figure 2:
   Increasing doses of SLC/TGFβ-LAP loaded on bifunctionally TCR- and TGFβR-activating anti-CD3/CD28/LAP antibody-coated beads significantly suppress and block the proliferation of T lymphocytes.
   The proliferation of activated human T lymphocytes is measured via the incorporation of exogenous radioactive thymidine. Thymidine incorporation is shown by T-cell-associated radioactivity in counts per minute (cpm). Blockage of T cell proliferation by active TGFβ cytokine only occurs when TCR/CD28 activating stimuli and TGFβ were presented to T cells together by the same bead surfaces (not shown).
Figure 3:
   Attenuation of T cell activation response by TGFβ1-LAP loaded on anti-CD3/CD28/LAP beads.
   A: TGFβ1-LAP on anti-CD3/CD28/LAP activating beads, shown in ng/ml final concentration, holds back bead-induced proliferation of human T lymphocytes in a dose-dependent manner, nearing a complete attenuation at highest doses. Proliferation was assayed by [3H]-thymidine incorporation, depicted in counts per minute (cpm). Data present averages and SEMs of 3 independent experiments with hexuplicate cultures each.
   B: Inactive LAP and TGFβ1-LAP were loaded onto anti-CD3/CD28/LAP beads in parallel titrations. TGFβ1-LAP on activating beads recapitulated dose-dependent silencing of T cell proliferative response to TCR/CD28 activation. Loading of LAP elicited significant inhibition of proliferation only at highest doses (n = 4). **, *** depict respective significant difference of p <0.01, 0.005 to 100% proliferation response elicited by anti-CD3/CD28/LAP beads.
   C: Western blots show that bead-induced tyrosine phosphorylation of TCR-proximal signaling proteins PLCy, ZAP-70, and LAT is attenuated by TGFβ1 presentation on anti-CD3/CD28/LAP beads. β-actin was probed as a loading control. Representative anti-phosphotyrosine blots of 3 independent experiments are shown.
   D: Gradual release of TGFβ1 cytokine by TGFβ1-LAP-loaded beads into the aqueous supernatant of ELISA wells.
Figure 4:
   Selective targeting of TGFβ1 into TCR/CD28 activation sites attenuates T cell proliferation response.
   A: TGFβ1-LAP, when presented to human T lymphocytes by anti-LAP- (gray triangles) or anti-LAP/CD28- (white squares) antibody-coated protein G beads, does not inhibit proliferative response elicited by separate anti-CD3/CD28 antibody-coated beads (n = 3). TGFβ1-LAP presented on anti-CD3/CD28/LAP beads (black circles) effectively held back proliferation (n = 4).
   B: Soluble TGFβ1, as latent TGFβ1-LAP (black circles) or active TGFβ1 cytokine (white squares), was present in equimolar amounts as TGFβ1-LAP for Figures 3A and 4A and did not effectively hold back T cell proliferation induced by activating anti-CD3/CD28 beads.
Figure 5:
   Attenuated proliferative response of bead-silenced T cells to TCR/CD28-activating culture dishes.
   T cells were incubated in round-bottom tubes with anti-CD3/CD28/LAP beads which were loaded with indicated amounts of TGFβ1-LAP. After A) 15 min and B) 24 h bead/T cell activation reactions were transferred onto tissue culture plates which were either left untreated (black bars) or were coated with anti-CD3 and anti-CD28 antibodies (gray bars) *, **, *** depict significant (p <0.05, 0.01, 0.005) attenuation of proliferation rates when T cells were preincubated with TGFβ1-LAP on activating beads.
   A: Transfer of 15 min-incubated bead/T cell conjugates to uncoated plates recapitulated dose-dependent attenuation of T cell proliferation by TGFβ1-LAP on activating beads. These T cells strongly responded to independent anti-CD3/CD28 stimulus on culture plates. This additional proliferative response was inhibited by initial TGFβ1-LAP-presentation in dose-dependent manner; down to ∼31% at highest loading of TGFβ1-LAP (n = 4).
   B: After 24 h of bead/T cell preincubation T cells responded with additional proliferation to plate-bound anti-CD3/CD28 antibodies. Proliferation on anti-CD3/CD28 plates, however, was restored to nearly the same rates with and without TGFβ1-LAP preincubation on beads; down to ∼85% at highest doses (n = 3).
Figure 6:
   Cytokine array of human pan T cell cytokine secretion-profiles.
   A: TCR/CD28 activating-antibodies on anti-CD3/CD28/LAP beads induced cytokine secretion (black bars) over non-activating control beads (white bars). Loading of TGFβ1-LAP on these TCR/CD28 activating beads suppressed activation-induced secretion of cytokines in a dose-dependent manner (gray bars).
   B: Cytokines which were secreted by resting T cells showed little induction following TCR-activation were not affected by TGFβ1 on TCR/CD28 activating beads. The cytokine array analysis was performed once and is detailed in Figure 9.
Figure 7:
   Spreading of T cells over TCR/CD28-activating bead surface.
   Isolated human resting T cells were conjugated with anti-CD3/CD28/LAP beads and incubated at 37°C for 45 min and analyzed by fluorescence microscopy. Outline of T cells was marked with fluorescent Alexa488 anti-actin mAb (please note weak binding of Alexa488 mAb to protein G beads), protein G beads with Alexa555 IgG1 mAb.
   A: Anti-CD3/CD28/LAP beads triggered spread of cells over bead surface leading to their partial or complete engulfment. Actin was depleted from the centre of bead/ T cell contact region and actin-rich lamellipodia formed at the periphery.
   B: TCR/CD28-mediated bead-engulfment by T cells and actin reorganization was suppressed in conjugates with TGFβ1-presenting activating beads. Scale bar =10 µm.
   C: Bar graphs indicate diameter of contact area of 23 (0 ng/ml TGFβ1) or 14 (20 ng/ml TGFβ1) bead/cell conjugates in two independent experiments. Boxes mark the 25% and 75% percentiles and error bars 5% and 95% percentiles of the contact secants. *** mark significant difference of p < 0.0001 in two-tailed Mann-Whitney testing. Please note that 5% and 25% percentiles in data sets of 0 ng/ml TGFβ1 cannot be graphed separately.
Figure 8:
   Viability of T cells is unchanged on presentation of TGFβ1-LAP on TCR/CD28-activating beads.
   Bead T cell conjugates were incubated for indicated times at 37°C and percentage of viable, propidium iodide-excluding cells was determined by FACS analysis.
Figure 9:
   Documentation; Cytokine array analysis.
   A: Arrangement of cytokine-trapping antibodies on array,
   B: Chemiluminescence film exposure,
   C: Semi-quantitative analysis of cytokine-chemiluminescence signals using software Kodak D1 3.6.

The present invention will be further illustrated in the following examples without being limited thereto.

### Examples

### Materials and Methods:

### Materials

Protein G-coated dynabeads were obtained from Novex Life technologies. Pan T cell isolation kit II was from Miltenyi Biotec. HIT3a anti-CD3 mAb and CD28.2 anti-CD28 mAb were from Becton Dickinson. Anti-LAP-TGFp mAb; latent TGFβ1; active TGFβ1 cytokine, cytokine array kit and TGFβ cytokine ELISA assay were from R&D Systems. Anti-phospho-PLCγ1 (Tyr783), anti-phospho-Zap-70 (Tyr319), and anti-phospho-LAT (Tyr191) were obtained from Cell Signaling Technology, anti-p-Actin monoclonal antibody from Sigma. HRP-conjugated donkey anti-mouse IgG or donkey anti-rabbit IgG (Dianova, Hamburg, Germany) were used as secondary antibodies. Ficoll was from Biochrome, [3H]-thymidine from MP Biomedicals, AIM-V culture medium from Invitrogen.

### Isolation of resting human T cells

Human peripheral blood mononuclear cells (PBMC) were isolated by Ficoll gradient centrifugation of heparinized blood collected from healthy volunteers. Human T cells were further purified by non-T cell depletion using the Pan T cell isolation kit II (Miltenyi Biotec). Purity of isolated pan T cells was ≥99%. Cells were washed twice and resuspended in serum-free AIM-V culture medium (Invitrogen). The study was approved by the local ethics committee. Blood donors gave written informed consent.

### Ligand-coating of beads, ELISA analysis, and preparation of anti-CD3/CD28 antibody-bound tissue culture plates

0.6 mg of protein G dynabeads were coated with 11 µg of antibody mix; anti-CD3, anti-CD28 and anti-LAP antibodies at 1:5:5 ratios; anti-LAP, anti-CD28 antibodies at 1:1 ratios, or anti-LAP antibodies alone. After wash in 2x1 ml PBS, 0.5% BSA beads were suspended in 800 µl PBS, 0.5% BSA for microscopy, ELISAs, array analysis, and proliferation assays or in 80 µl PBS, 0.5% BSA for preparing Western blot samples. Antibody-coated beads in required minimal volumes were incubated with TGFβ1-LAP to achieve final concentrations of 0, 0.1, 0.2, 0.5, 1, 2, and 5 ng/4 µl of bead suspension. Anti-CD3/CD28/LAP beads for Western blot samples were loaded to 20 ng TGFβ1-LAP /4 µl suspension. Bead/TGFβ1-LAP mix was incubated over night on a rotating wheel at 4°C, suspended in 1 ml of PBS, 0.5% BSA in ice and harvested with a magnet. Pelleted beads were then suspended in PBS, 0.5% BSA, retaining above concentrations of beads and bead-bound TGFβ1-LAP.

ELISA: 3 µg anti-CD3/CD28/LAP protein G beads were loaded with 5 ng latent TGFβ1-LAP, transferred into 100 µl AIMV medium in commercial TGFβ-ELISA wells (BD Life Technologies) and incubated for 1 h (gray bars) and 24 h (black bars) at 37°C. 10⁵ T cells were present in some wells. Plates were developed according to manufacturer's instruction in quadruplicate wells per condition.

Plastic tissue culture plates were coated with goat anti-mouse secondary antibodies (Dianova) 1:100 in PBS 50 µl/well over night at 4°C, washed in PBS, and then incubated for 1 h with hybridoma supernatants of anti-CD3 (Okt3) and anti-CD28 (CD28.2) monoclonal antibodies; 1:100 in PBS.

*Proliferation-, cytokine secretion-assay, viability assessment and Western blot* 10⁵ T cells were incubated with 4 µl (2x10⁵) of respective ligand-coated beads in round bottom wells of 96 well microtiter plates (Nunc) with 100 µl of serum-free AIM-V medium (six wells per condition). Proliferation was assessed after 3 days by measuring [³H]-thymidine [³H-TdR] incorporation. [³H]-thymidine was added at 0.2 µCi/well for 3 h. At the end of the incubation period, cells were harvested and radioisotope incorporation was measured as index of lymphocyte proliferation using a betaplate liquid scintillation counter (MicroBeta, Wallac).

Cytokine secretion profile following 3 days of cell/bead incubation was determined from 10x100 µl AIM-V T cell supernatant per condition and was assayed using commercial cytokine array kit (R&D) according to manufacturer's instruction. Semi-quantitative analysis was performed by determining the background-subtracted sum intensity for each dot on the membrane using Kodak D1 3.6 software. Densitometry values of spots on different membranes were normalized using the results for the positive controls.

Data were analyzed, graphed, and subjected to statistical analyses using Graphpad Prism software. *, **, *** depict statistical significance p <0.05, 0.01, 0.005, respectively assessed by one way Anova testing.

Cell viability was determined in parallel cell cultures by propidium iodide (PI) staining. After 4 h, 24 h, and 72 h of incubation with respective activating beads, cells were incubated with PI/RNase staining buffer (BD Pharmingen) for 15 min and percentage of PI-excluding cells was subsequently determined by FACS according to manufacturer's instructions.

For Western blot analyses whole cell lysates were prepared from 10⁶ T cells in 20 µl AIM-V medium. T cells were incubated with 4 µl (2x10⁶) TCR/CD28 activating beads with and without TGFβ1-LAP coating, incubated at 37°C for 0, 1, 5, 30 min, and lysed in a final volume of 100 µl 1 x SDS sample buffer. Proteins, 20 µl sample per lane, were separated in 10% SDS-polyacrylamide gel and transferred to Hybond N membranes by semi-dry blotting. Specific binding was visualized by chemiluminescence (Thermo Scientific, Rockford, USA).

### Microscopy

Bead/cell conjugates were incubated at 37°C for 45 min and adhered to poly-L-lysine coated microscopy slides. After 15 min fixation with 2% PFA cells were permeabilized for 10 min with 0.2% TX100 in PBS, washed in PBS/1% BSA for 30 min. Actin of cells was labeled with an Alexa488 conjugated anti-actin antibody (BD, 558623) and protein G beads were marked with Alexa555-labelled IgG1 mAb (Cell Signaling). Fluorescence images were recorded with a CCD camera (Apogee, KX4) mounted to a fluorescence microscope (Leica, DM IRE2) using Alexa488 (Omega Filters, XF116-2) and Alexa555 (Omega Filters, XF111-2) channel settings at 63x magnification (Leica, Oil, NA 1.40, 506206). Maximal lengths of the secants in 14 (20 ng TGFβ1-LAP) and 23 (0 ng TGFβ1-LAP) at the respective bead/cell contact zones was measured. *** mark significant inhibition (p < 0.0001) of bead engulfment by TGFβ1-LAP-loading in two tailed Mann-Whitney testing.

### Example 1:

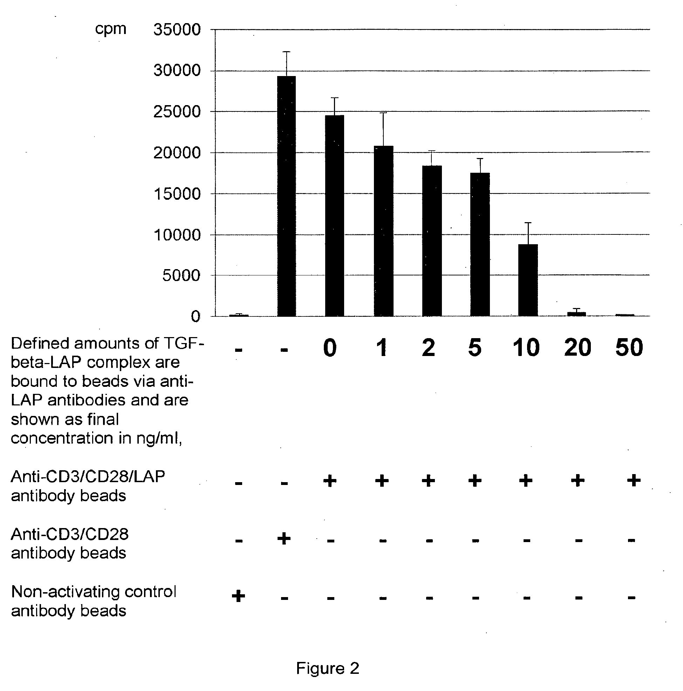

The proliferation of activated human T lymphocytes has been measured via the incorporation of exogenous radioactive thymidine which is incorporated into the cells during cell division. As can be seen in Figure 2, increasing doses of TGFβ presentation by bifunctional TCR- and TGFβR-activating surfaces significantly suppresses the proliferation of T lymphocytes. At a dose of 20 ng/ml final concentration of TGB-β-LAP presentation, T cell proliferation is completely blocked. Suppression of T cell proliferation does not occur when the T cell activating stimulus and TGFβ presentation are effected from separate surfaces (data not shown).

### Example 2:

### Presentation of TGFβ1 on TCR/CD28-activating beads silences T cell activation responses.

Isolated resting human T lymphocytes were activated via TCR/CD28 by conjugation with protein G-coated microbeads which were coated with anti-CD3 and anti-CD28 mAbs (monoclonal antibodies) and, additionally, with mAb against the LAP subunit of latent TGFβ-LAP complex. Defined amounts of TGFβ1-LAP were loaded onto anti-CD3/CD28/LAP beads via anti-LAP antibodies. TGFβ1-LAP-loading silenced proliferative response of T cells to bead-bound anti-CD3 and anti-CD28 mAbs in strictly dose-dependent manner; first evident around 2 ng/ml final concentration and nearing completion at 50 ng/ml (Figure 3A).

Contribution of steric impediment of antibody access to T cells by bead-bound TGFβ1-LAP to attenuation of T cell proliferation response was estimated. In parallel experiments, TCR/CD28-activating beads were loaded with inactive LAP and with latent TGFβ1-LAP. Inactive LAP, when loaded on T cell-activating anti-CD3/CD28/LAP beads, partially inhibited proliferation at highest doses (Figure 3B) indicating that steric impediment of activating antibody access contributes to the inhibitory effect. This, however, can be discriminated from strictly dose-dependent and stringent antiproliferative activity of TGFβ1-LAP on activating beads.

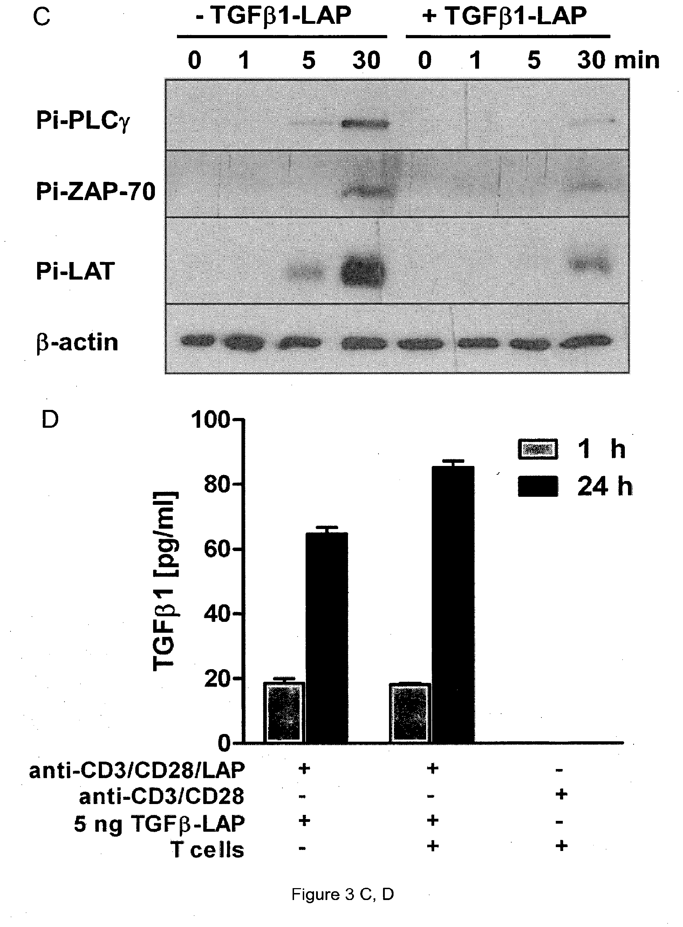

It was then assessed whether presentation of TGFβ1-LAP on anti-CD3/CD28/LAP beads also attenuated early response of tyrosine phosphorylation of TCR-proximal signaling proteins. Western blots of whole cell lysates of TCR/CD28 activating bead-stimulated T cells were performed over a time course of 30 mins, 37°C incubation. These blots reiterated TCR triggering-induced tyrosine phosphorylation of tyrosine kinase ZAP-70, transmembrane Linker for Activation of T cells (LAT) and phospholipase C γ (PLCγ), with respective Mw at 70 kD, 36 kD and 110 kD (Figure 3C). Importantly, when TGFβ1-LAP was presented by TCR/CD28-activating beads, tyrosine phosphorylation of ZAP-70, LAT, and PLCγ was reduced. These data suggest that silencing of T cell activation responses upon TGFβ1-LAP presentation on TCR/CD28-activating beads commences with attenuation of early TCR-proximal tyrosine phosphorylation.

Holding back T cell activation response by local presentation of latent TGFβ1-LAP implied mechanisms which release active TGFβ1-cytokine toward TCR/CD28 activation domains. ELISA analysis of bead supernatants indicated spontaneous release of TGFβ1-cytokine from beads at a slow rate (Figure 3D) which may be sufficient for the T cell-silencing mechanism described here. Presence of T cells elicited an additional ∼30% release of TGFβ1 at 24 h 37°C incubation. Association/dissociation kinetics of TGFβ-LAP complexes and of TGFβ with TGFβRI and II were comprehensively characterized in plasmon resonance studies, unraveling rates of spontaneous slow dissociation and rapid rebinding of TGFβ1 to LAP, consistent with our results. In our ELISA assays bead-bound non-complexed LAP molecules compete with TGFβ-capturing ELISA antibodies for binding free TGFβ1 cytokine. Moreover, T cellular activities partially contribute to latent TGFβ1-LAP activation. In conclusion, ELISAs demonstrated gradual release of active TGFβ1 cytokine from beads but could not estimate quantities and rates in T cell/bead-conjugates.

### Example 3:

### TGFβ and TCR/CD28 signals cooperate in common plasma membrane domains in order to silence T cell proliferative response.

We next queried the requirement of simultaneously presenting TCR/CD28 activating stimulus and TGFβ1 activity on the same bead for holding back T-cell proliferation (Figure 4). This was addressed by presenting anti-CD3/CD28 stimulus and TGFβ1-LAP on separate beads (Figure 4A). TGFβ1-LAP was presented by protein G beads which were either coated with anti-LAP antibodies alone or with anti-LAP and anti-CD28 antibodies at a 1:1 ratio in order to enhance contact of anti-LAP beads with T cells. Importantly, both ways of presenting TGFβ1-LAP to T cells had little influence on proliferative response induced by anti-CD3/CD28 beads. This indicated that stringent silencing of T cell proliferative response only occurred when TCR/CD28 activating stimuli and TGFβ1-LAP were presented to T cells together on the same bead.

TGFβ1 was presented to T cells as soluble latent TGFβ1-LAP complex or as soluble active TGFβ1 cytokine and elicited little antiproliferative effect on T cells which were activated by anti-CD3/CD28 protein G beads (Figure 4B). It is particularly important to emphasize that, at this specific setting of T cell activation, soluble active TGFβ1 cytokine could not substitute for the lack of TGFβ1 presentation on T cell activating beads in blocking activation response, suggesting that TCR/CD28- and TGFβ-signals integrate and cooperate in common T cell plasma membrane domains and then provide signals which silence T cell activation response. Interestingly, MHC class II expression identifies a distinct population of human Tregs which mediates early suppression of effector T cell activation responses on direct cell/cell contact. It is tempting to speculate that silencing beads mimic suppressive activities of this MHC class II + Treg population in the human system.

### Example 4:

### Diminished proliferative response of bead-silenced T cells to TCR/CD28-activating culture plates.

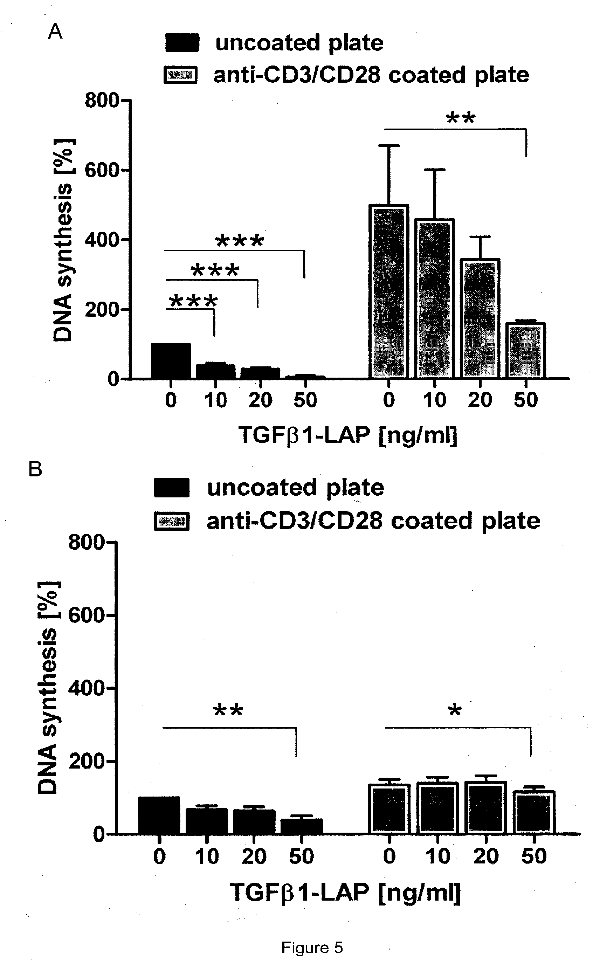

It was observed that TGFβ1 elicits potent inhibition of T cell proliferative responses when presented in physical proximity of activating TCR/CD28 stimuli. It was sought to characterize response of these TGFβ1-silenced T cells when activated by fresh, independent TCR/CD28 stimulus (Figure 5). First, T cells were silenced for 15 min at 37°C with TGFβ1-LAP on anti-CD3/CD28/LAP beads (Figure 5A). T cell/bead conjugates were then transferred to tissue culture plates which were either left untreated or were coated with anti-CD3 and anti-CD28 antibodies. After 3 days on plates, T cell proliferation rates were determined. [³H]-thymidine incorporation was used to measure T cell proliferation rates. On transfer to uncoated non-activating plates proliferation was induced by activating beads without TGFβ1-LAP loading at 15635 ± 9310 cpm SEM and was taken as 100% proliferative response value. Variation and relatively low proliferation rates may be caused by variable loss of T cell/bead contact on transfer. Dose-dependent and stringent antiproliferative action of bead-bound TGFβ1-LAP, however, was always recapitulated. Transfer of 15 min-incubated T cells/beads to anti-CD3/CD28-coated tissue culture plates strongly enhanced T cell proliferation rates. Proliferative response to plate-bound anti-CD3/CD28-activating stimulus was significantly attenuated to ∼31% by TGFβ1-LAP presentation on initial activating beads. These results indicate that T cells which were silenced by anti-CD3/CD28/LAP bead-presented TGFβ1 for 15 min exhibited a reduced response to fresh TCR/CD28 activating surface.

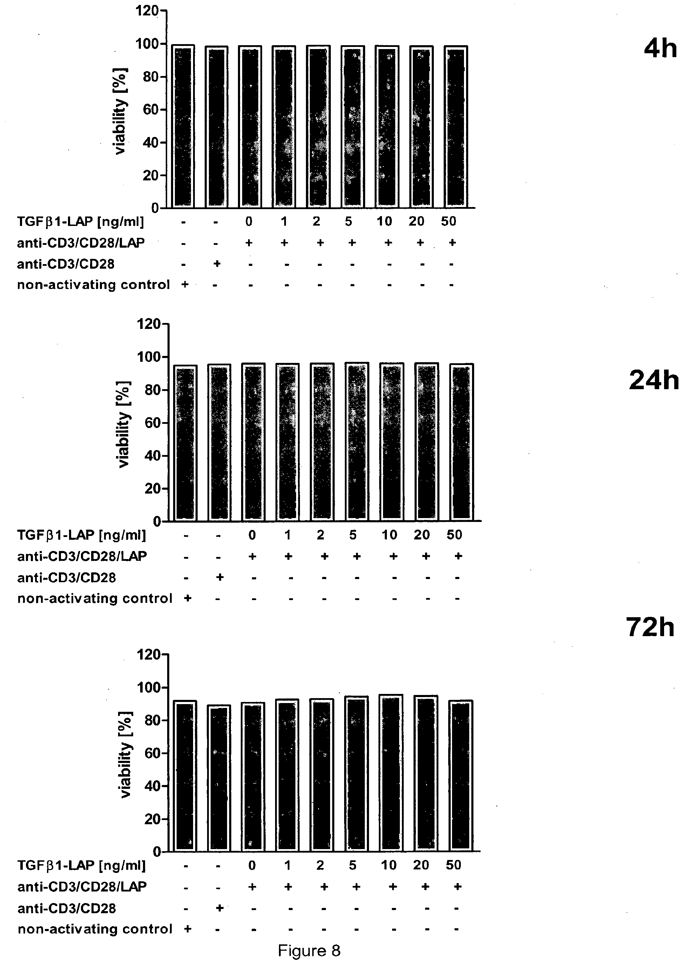

Next, initial bead/T cell activation reactions were incubated for 24 h before transfer to tissue culture plates (Figure 5B): On transfer to uncoated plates, bead-induced proliferation without TGFβ1-LAP was measured at 47881 ± 12792 cpm SEM. Again proliferation of T cells was inhibited by TGFβ1-LAP loaded on TCR/CD28-activating beads (Figure 5B). Here, however, TGFβ1-mediated suppression of T cell proliferation was less pronounced than for bead/T cell conjugates after 15 min preincubation in Figure 5A. This suggested that after 24 h of preincubation, silencing activity of TGFβ1-LAP-loaded TCR/CD28-activating beads was reduced, and that silenced T cells reverted to be receptive to renewed proliferative stimulus by fresh contact with bead-bound anti-CD3/CD28 antibodies on transfer. Indeed, 24 h-preincubated T cells responded to anti-CD3 and anti-CD28 on plates with little difference in proliferation rates whether the 24 h-preincubation was performed with or without TGFβ1-LAP on activating beads. In conclusion, silencing of T cells by TGFβ1-LAP-loaded beads was reverted on longer incubations and thus did not reflect a long-lasting T cell anergic state or T cell death. Figure 8 documents that T cell viability is unaltered under all experimental conditions.

### Example 5:

### A range of T cell functions is attenuated when TGFβ1 is presented on activating beads.

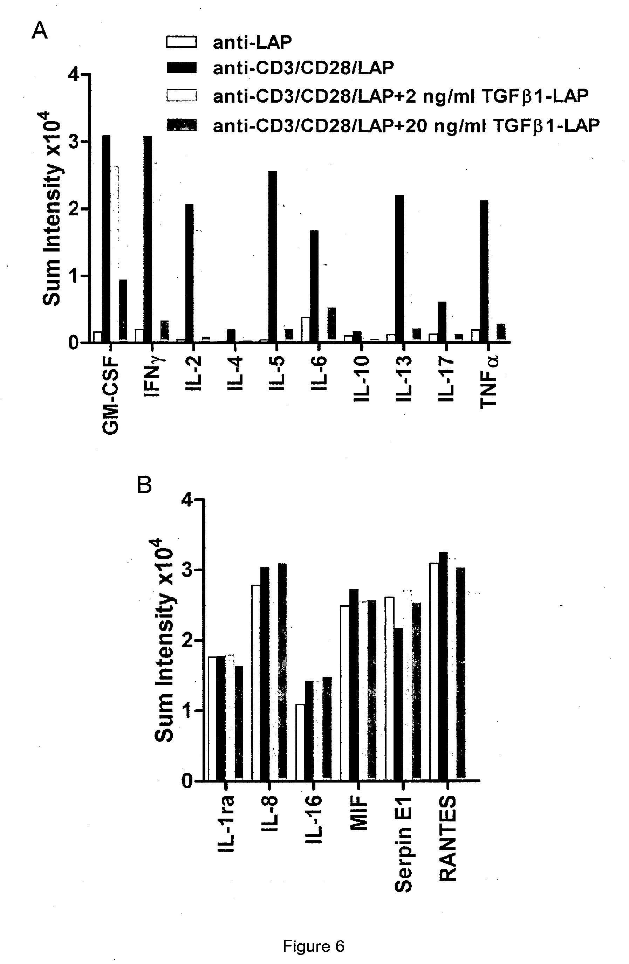

A pan human T cell cytokine array was used to characterize the range of T cell effector responses which were silenced by TGFβ1-presentation on TCR/CD28-activating beads (Figure 6). As expected, activating beads alone induced secretion of cytokines IFN-γ, IL-2, IL-4, IL-10, IL-17, TNF-α. Consistent with attenuated TCR-proximal tyrosine phosphorylation and T cell proliferation, bead-bound TGFβ1-LAP held back secretion of these cytokines in a dose-dependent manner to nearly baseline levels (Figure 6A). T cell effector functions which were attenuated by TGFβ1-LAP on activating beads included inflammatory responses (TNF-α), T_{H}1 and T_{H}2 functions (IFN-γ, IL-4), T_{H}17 functions (IL-17) and regulatory T cell activities (IL-10). Other cytokines, like IL-8 and IL-16 were secreted by resting T cells and showed little induction following TCR-activation (Figure 6B). Consistently, secretion of these cytokines was not altered by TGFβ1 presentation on TCR/CD28 activating beads. Cytokine array analysis was performed once and is fully documented in Figure 9.

### Example 6:

### TGFβ1-presentation hinders spreading of T cells over TCR/CD28-activating bead surface.

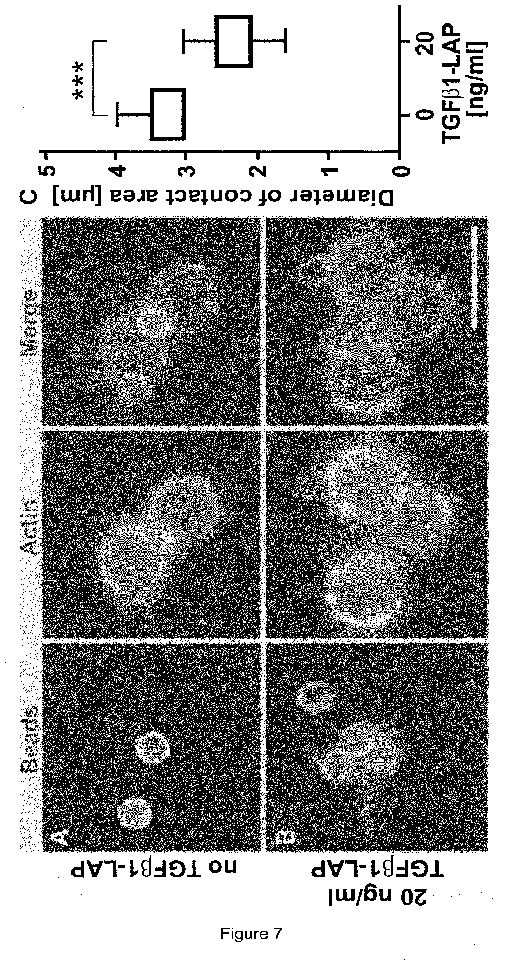

Next, spreading of T cells over anti-CD3/CD28/LAP bead surfaces with and without TGFβ1-LAP loading was characterized by epifluorescence microscopy and statistical analysis (Figure 7). Bead/T cell conjugates, after 45 min incubation at 37°C, were stained with Alexa488 fluorescent anti-actin mAb to visualize the circumference of T cells, and protein G beads were additionally marked with Alexa555 mouse IgG1. Consistent with previous reports, anti-CD3/CD28/LAP antibodies alone induced T cell-spreading over the surface of TCR/CD28-activating beads leading to their engulfment (Figure 7A) and actin-containing structures formed at the edges of the beads while actin was depleted from the central region of bead-cell contact. Importantly, loading of TGFβ1-LAP on activating beads significantly inhibited engulfment of TCR/CD28-activating beads (Figure 7B, C). Inhibition of T cell spreading over TCR-activating surfaces was reported to attenuate T cell activation response.

### Discussion:

Using ligand-presenting beads, TGFβ1 and TCR/CD28-activating signals were directed into defined plasma membrane domains of T cells. Selective targeting of TGFβ1 cytokine into TCR/CD28 signaling plasma membrane domains held back early response of TCR-proximal tyrosine phosphorylation and bead engulfment at activation sites. Consequently, downstream induction of proliferation and cytokine secretion were stringently attenuated. After extended incubation with TGFβ1-presenting beads, silenced T cells became receptive again to activation by renewed TCR/CD28-stimuli, indicating that the unresponsive state of T cells was reverted and did not reflect long-lasting anergy or decrease in T cell viability. These findings outline a new strategy of physically linking TGFβ1 and TCR-activating functions for the treatment of disease and pathological conditions which are caused by unwanted T cell activity.

## Claims

1. A bifunctional agent that is suitable for suppressing the activation of T lymphocytes, said agent having the general structure 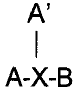 wherein
A is an antibody, antibody fragment or antibody mimetic directed against CD3,
A' is an antibody, antibody fragment, or antibody mimetic directed against CD28,
B is an antibody, antibody fragment, or antibody mimetic directed against latency-associated peptide (LAP) of the TGFβ-LAP Small Latent Complex, wherein said antibody, antibody fragment or antibody mimetic is loaded with exogenous TGFβ-LAP complexes, and
X is selected from the group consisting of a bead, a magnetic bead, a polymeric matrix, and a solid support,
wherein said agent comprises more than one individual molecule of the types A, B, and A',
wherein said antibodies, antibody fragments or antibody mimetics and the TGFβ-LAP complexes are present in a ratio of 0.5 to 1.5 parts A, 2.5 to 7.5 parts A', 2.5 to 7.5 parts B, and 0.02 to 10 parts TGFβ-LAP complexes, and wherein said antibody mimetics are selected from the group consisting of single-chain variable fragments (scFv), single-domain antibodies, affibodies, anticalins, DARPins, monobodies, and peptide aptamers.

2. The agent according to claim 1, wherein said TGFβ-LAP complexes are TGFβ1-LAP complexes.

3. The agent according to claim 1 or claim 2, wherein X is a bead.

4. The agent according to claim 3, wherein a single bead comprises 1 × 10⁷ to 1 × 10⁸ individual TGFβ-LAP complexes.

5. The agent according to any one of claims 1 to 4 for use in medicine.

6. The agent according to any one of claims 1 to 4 for use in the prevention or treatment of a disease or condition that is associated with excessive and/or uncontrolled T lymphocyte activation.

7. The agent for use according to claim 6, wherein the disease or condition that is associated with excessive and/or uncontrolled T lymphocyte activation is a disease or condition selected from the group consisting of autoimmune diseases, such as diabetes mellitus type I, multiple sclerosis (MS), and rheumatoid arthritis; inflammatory diseases, such as inflammatory bowel diseases, Morbus Crohn, and ulcerative colitis; transplant rejection; allograft rejection; graft-versus-host disease; and allergies.

8. An *in vitro* method for the suppression of the activation of T lymphocytes, said method comprising the step of incubating the T lymphocytes with an agent according to any one of claims 1 to 4.

9. The method according to claim 8, wherein the suppression is a complete blocking of T lymphocyte activation.

## Patentansprüche

1. Bifunktionelles Mittel, das geeignet ist, die Aktivierung von T-Lymphozyten zu unterdrücken, wobei das Mittel die allgemeine Struktur 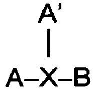 aufweist,
wobei
A ein Antikörper, Antikörperfragment oder Antikörpermimetikum ist, der/das gegen CD3 gerichtet ist,
A' ein Antikörper, Antikörperfragment oder Antikörpermimetikum ist, der/das gegen CD28 gerichtet ist,
B ein Antikörper, Antikörperfragment oder Antikörpermimetikum ist, der/das gegen *latency-associated peptide* (LAP) des TGFβ-LAP *Small Latent-*Komplexes gerichtet ist, wobei der Antikörper, das Antikörperfragment oder Antikörpermimetikum mit exogenen TGFβ-LAP-Komplexen beladen ist und
X ausgewählt ist aus der Gruppe, bestehend aus einem Mikrokügelchen, einem magnetischen Mikrokügelchen, einer polymeren Matrix und einem festen Träger,
wobei das Mittel mehr als ein individuelles Molekül der Typen A, B und A' umfaßt,
wobei die Antikörper, Antikörperfragmente oder Antikörpermimetika und die TGFβ-LAP-Komplexe in einem Verhältnis von 0,5 bis 1,5 Teilen A, 2,5 bis 7,5 Teilen A', 2,5 bis 7,5 Teilen B und 0,02 bis 10 Teilen TGFβ-LAP-Komplexe vorhanden sind und
wobei die Antikörpermimetika ausgewählt sind aus der Gruppe, bestehend aus einzelkettigen variablen Fragmenten (*single-chain variable fragments;* scFv), Eindomänenantikörpern, Affibodies, Anticalinen, DARPinen, Monobodies und Peptidaptameren.

2. Mittel nach Anspruch 1, wobei die TGFβ-LAP-Komplexe TGFβ1-LAP-Komplexe sind.

3. Mittel nach Anspruch 1 oder Anspruch 2, wobei X ein Mikrokügelchen ist.

4. Mittel nach Anspruch 3, wobei ein einzelnes Mikrokügelchen 1 × 10⁷ bis 1 × 10⁸ individuelle TGFβ-LAP-Komplexe umfaßt.

5. Mittel nach einem der Ansprüche 1 bis 4 zur Verwendung in der Medizin.

6. Mittel nach einem der Ansprüche 1 bis 4 zur Verwendung in der Vorbeugung oder Behandlung einer Erkrankung oder eines Zustands, die/der mit übermäßiger und/oder unkontrollierter Aktivierung von T-Lymphozyten in Verbindung steht.

7. Mittel zur Verwendung nach Anspruch 6, wobei die Erkrankung oder der Zustand, die/der mit übermäßiger und/oder unkontrollierter Aktivierung von T-Lymphozyten in Verbindung steht, eine Erkrankung oder ein Zustand ist, ausgewählt aus der Gruppe, bestehend aus Autoimmunerkrankungen, wie Diabetes mellitus Typ I, multiple Sklerose (MS) und rheumatische Arthritis; entzündlichen Erkrankungen, wie entzündliche Darmerkrankungen, Morbus Crohn und ulcerative Colitis; Transplantatabstoßung; Allograftabstoßung; Graft-versus-Host-Erkrankung und Allergien.

8. *In vitro* Verfahren für die Unterdrückung der Aktivierung von T-Lymphozyten, wobei das Verfahren den Schritt des Inkubierens der T-Lymphozyten mit einem Mittel nach einem der Ansprüche 1 bis 4 umfaßt.

9. Verfahren nach Anspruch 8, wobei die Unterdrückung eine vollständige Blokkierung der Aktivierung von T-Lymphozyten ist.

## Revendications

1. Agent bifonctionnel qui est adapté pour supprimer l'activation des lymphocytes T, ledit agent ayant la structure générale 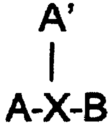 dans lequel
A est un anticorps, un fragment d'anticorps ou un mimétique d'anticorps dirigé contre CD3,
A' est un anticorps, un fragment d'anticorps ou un mimétique d'anticorps dirigé contre CD28,
B est un est un anticorps, un fragment d'anticorps ou un mimétique d'anticorps dirigé contre le peptide associé à la latence (LAP) du petit complexe latent TFGβ-LAP, dans lequel ledit anticorps, fragment d'anticorps ou mimétique d'anticorps est chargé de complexes TGFβ-LAP exogènes, et
X est sélectionné dans le groupe constitué d'une bille, d'une bille magnétique, d'une matrice polymère, et d'un support solide,
dans lequel ledit agent comprend plus d'une molécule individuelle des types A, B et A',
dans lequel lesdits anticorps, fragments d'anticorps ou mimétiques d'anticorps et les complexes TGFβ-LAP sont présents en un rapport de 0,5 à 1,5 partie d'A, 2,5 à 7,5 parties d'A', 2,5 à 7,5 parties de B et 0,02 à 10 parties de complexes TGFβ-LAP, et dans lequel lesdits anticorps mimétiques sont sélectionnés dans le groupe constitué de fragments variables de chaîne unique (scFv), d'anticorps à domaine unique, d'afficorps, d'anticalines, de DARPines, de monocorps, et d'aptamères peptidiques.

2. Agent selon la revendication 1, dans lequel lesdits complexes TGFβ-LAP sont des complexes TGFβ1-LAP.

3. Agent selon la revendication 1 ou la revendication 2, dans lequel X est une bille.

4. Agent selon la revendication 3, dans lequel une bille unique comprend 1 x 10⁷ à 1 x 10⁸ complexes TGFβ-LAP individuels.

5. Agent selon l'une quelconque des revendications 1 à 4 pour son utilisation en médecine.

6. Agent selon l'une quelconque des revendications 1 à 4 pour son utilisation dans la prévention ou le traitement d'une maladie ou d'une affection associée à une activation excessive ou incontrôlée des lymphocytes T.

7. Agent pour son utilisation selon la revendication 6, dans lequel la maladie ou l'affection associée à une activation excessive et/ou incontrôlée des lymphocytes T est une maladie ou une affection sélectionnée dans le groupe constitué des maladies auto-immunes, telles que le diabète sucré de type I, la sclérose en plaques (SEP), et la polyarthrite rhumatoïde ; des maladies inflammatoires, telles que les affections abdominales inflammatoires, la maladie de Crohn, et la rectocolite hémorragique ; le rejet de greffe ; le rejet d'allogreffe ; la maladie du greffon contre l'hôte ; et les allergies.

8. Procédé *in vitro* de suppression de l'activation des lymphocytes T, ledit procédé comprenant l'étape d'incubation des lymphocytes T avec un agent selon l'une quelconque des revendications 1 à 4.

9. Procédé selon la revendication 8, dans lequel la suppression est un blocage complet de l'activation des lymphocytes T.
